Europäisches Patentamt

European Patent Office

Office européen des brevets

(11) Publication number: **0 450 480 B1**

## EUROPEAN PATENT SPECIFICATION

(45) Date of publication of patent specification: **21.06.95** (51) Int. Cl.6: **C07K 14/655**, A61K 38/31

(21) Application number: **91104845.2**

(22) Date of filing: **27.03.91**

The file contains technical information submitted after the application was filed and not included in this specification

(54) **Somatostatin Analogues.**

(30) Priority: **06.04.90 US 505501**

(43) Date of publication of application:
**09.10.91 Bulletin 91/41**

(45) Publication of the grant of the patent:
**21.06.95 Bulletin 95/25**

(84) Designated Contracting States:
**AT BE CH DE DK ES FR GB GR IT LI LU NL SE**

(56) References cited:
**EP-A- 203 031**

**PROCEEDINGS OF THE NATIONAL ACADEMY OF SCIENCES OF USA, vol 86, no. 16, 1989 Washington US pages 6318-6322. S Bajusz C.S. "Highly potent analogues of LHRH containing D-Phenylalanine nitrogen mustard in position 6"**

**PROCEEDINGS OF THE NATIONAL ACADEMY OF SCIENCES OF USA, vol. 86, no,16, 1989 Washington US, pages 6313-6317, S. Bajusz C.S. "Highly potent metallopeptide analogues of LHRH"**

(73) Proprietor: **The Administrators of The Tulane Educational Fund**
**1430 Tulane Avenue**
**New Orleans**
**Louisiana 70112 (US)**

(72) Inventor: **Schally, Andrew V.**
**5025 Kawanee Ave**
**Metairie, LA 70006 (US)**
Inventor: **Janaky, Tamas**
**Hullum u. 7sz, II/10**
**H-6721 Szeged (HU)**
Inventor: **Cai, Ren Zhi**
**7024 Glenn Street**
**Metairie LA 70003 (US)**

(74) Representative: **Dr. Fuchs, Dr. Luderschmidt Dr. Mehler, Dipl.-Ing. Weiss Patentanwälte**
**Postfach 46 60**
**D-65036 Wiesbaden (DE)**

## Description

FIELD OF THE INVENTION

The present invention is directed to novel peptides having influence on growing of cancerous tumors in humans. More specifically, the present invention relates short, cyclic analogues of somatostatin which contain cytotoxic moieties, inhibit the secretion of the pituitary growth hormone and have antineoplastic effect; salts thereof, and to pharmaceutical compositions and methods of use pertaining to these analogues.

RELATED APPLICATIONS

This application is a continuation in part of our pending patent application Serial No. 07/404,667, filed 09/07/1989.

BACKGROUND OF THE INVENTION

The present invention relates to novel short, cyclic somatostatin analogues which contain cytotoxic moieties, have influence on the release of pituitary growth hormone in mammals and have antineoplastic effects. Tetradecapeptide somatostatin (SS-14) with structure of

```
 ┌─────────────────────────────────────────────────────────────┐
Ala-Gly-Cys-Lys-Asn-Phe-Phe-Trp-Lys-Thr-Phe-Thr-Ser-Cys
 1    2    3    4    5    6    7    8    9   10   11   12   13   14
```

is an inhibitor of growth hormone release and has subsequently been shown to have a broad spectrum of inhibiting properties, namely on the secretion of pancreatic insulin and glucagon. Conformational analysis and structure-function studies on somatostatin analogues indicate that the sequence required for biological activity consists of the $\beta$-turn fragment Phe-Trp-Lys-Thr corresponding to the residues 7-10 of somatostatin (D. Veber et al., Nature (London) 280, 512 (1979) and D. Veber et al., Nature (London) 292, 55 (1981)). Many short, cyclic analogues of somatostatin have been developed in the search for compounds with selective, enhanced and prolonged activity. Veber et al. have reported the synthesis and marked inhibitory activity of cyclic hexapeptide analogues obtained by replacing 9 of the 14 amino acids of somatostatin with a single proline or with N-MeAla (cyclo[Pro-Phe-D-Trp-Lys-Thr-Phe] and cyclo[N-MeAla-Phe-D-Trp-Lys-Thr-Phe]). Additionally, the same group synthesized a new hexapeptide, cyclo(N-MeAla-Tyr-D-Phe-Lys-Val-Phe), with high activity (D. Veber et al., Life Sci. 34, 1371 (1984)). Bauer et al. (Life Sci. 31, 1133 (1982)) synthesized another series of highly potent octapeptide analogues of somatostatin. Their most active analog was D-Phe-Cys-D-Trp-Lys-Thr-Cys-Thr-OL. We have developed nearly 300 octapeptide amide analogues related to their compound (R.-Z. Cai et al., Proc. Natl. Acad. Sci. USA 83, 1896 (1986) and R.-Z. Cai et. al., Proc. Natl. Acad. Sci. USA 84, 2502 (1987) and ). Two of these compounds [D-Phe-Cys-Tyr-D-Trp-Lys-Val-Cys-Thr-NH$_2$ and D-Phe-Cys-Tyr-D-Trp-Lys-Val-Cys-Trp-NH$_2$ proved to be more than 100 times more potent than somatostatin in test for inhibition of GH release, and showed prolonged duration of action.

Various studies demonstrated inhibitory effects of somatostatin in patients with acromegaly, endocrine pancreatic tumors such as insulinomas and glucagonomas, ectopic tumors like gastrinomas, and VIP producing tumors. However, somatostatin is of little therapeutic value in cancer therapy because of its multiple actions and the short duration of its antisecretory effects; half-life in circulation being about 3 min (A.V. Schally et al., Annu. Rev. Biochem. 47, 89 (1978)). The work of Veber et al. (1979-1984) was apparently aimed at producing analogues for therapy of diabetes Type I, and their analogues did not reveal antitumor activities in various animal tumor models (A.V. Schally et al., Proc.Soc. Exp. Biol. Med., 175, 259 (1984)). Bauer's analog (Sandostatin) was subjected to careful clinical evaluation (P. Marbach et al., (1985) in: Proc. Symp. Somatostatin, Montreal, Canada, 1984, Eds. Y.C. Patel & G.S. Tannenbaum, Elsevier, Amsterdam, p. 339). It seems to be promising therapeutic tool in the medical treatment of acromegaly, metastatic endocrine pancreatic and gastrointestinal tumors, such as insulinomas, glucagonomas, gastrinomas, VIPomas and carcinoid tumors (A.V. Schally et al., in:"Neural and Endocrine Peptides and Receptors", Ed. T.W. Moody, Plenum Publishing Corp., New York, 1986, p. 73).

Modern somatostatin analogues have been indicated as having utility for the treatment of some tumors, as well as adjuncts to therapy with LH-RH analogues in breast cancer (A.V. Schally, Cancer Res., 48, 6977 (1988)).

Ideal anticancer drugs would theoretically be those that eradicate cancer cells without harming normal cells. Hormones carrying antineoplastic agents would solve the problem by achieving more efficiently targeted chemotherapy of receptor-containing tumors. A hormonal analog which contains a cytotoxic group incorporated into or linked to the sequence of the hormone could possibly be targeted and therefore more selective in killing cancerous tissue bearing hormone receptors. An ideal mechanism of action of hormone-drug conjugates would be their binding to a cell membrane receptor, followed by internalization of the hybrid molecules and release of the drugs or their biologically active derivatives from the carrier hormone in the endosomes or secondary lysosomes. The released substances would then pass across the membrane of the vesicles into the cytosol and reach their final target sites. For the conjugates to be effective by this mechanism, the bond between the drug and hormone must be stable before internalization of conjugates into the target tumor cells but should be effectively cleaved after this internalization.

High or low affinity somatostatin receptors have been found in various normal human tissues, human brain and pituitary tumors, hormone producing gastrointestinal tumors, breast, pancreatic, prostate and ovarian cancers (D. Reichlin, N. Engl. J. Med., 309, 1495 (1983), A.V. Schally, Cancer Res., 48, 6977 (1988). It has been demonstrated (Viguerie, N. et al., Am. J. Physiol. 252: G 535-542, 1987), that receptor-bound somatostatin is internalized and degraded by pancreatic acini.

According to this concept, somatostatin analogues containing cytotoxic radicals could exert the direct or indirect antitumor effects of somatostatin analogues and, at the same time, could serve as carriers for the chemotherapeutic agents. Since such peptides can bind to specific receptors, this can provide some target selectivity for the chemotherapeutic compound and make it "cell specific" decreasing its nonspecific toxicity. After internalization, these hybrid compounds could interfere with cellular events causing death of cells.

There are several compounds among the clinically used anticancer drugs which have the possibility of coupling to a carrier peptide molecule. The coupling could be carried out through the suitable modified functional group of the cytotoxic moiety and the free amino- or carboxyl-group of a peptide.

Alkylating agents used in the treatment of cancer have basically nonselective mechanism of action. They act by exerting cytotoxic effects via transfer of their alkyl groups to various cell constituents. Alkylation of DNA within the nucleus probably represents the major interactions that lead to cell death. However, under physiologic conditions, they can alkylate all cellular nucleophiles such as ionized carboxylic and phosphoric acid groups, hydroxyl groups, thiols and uncharged nitrogen moieties. Nitrogen mustards (chlorambucil, cyclophosphamide, melphalan and nitrogen mustard) are among the oldest anticancer drugs in clinical use. They spontaneously form tricyclic aziridinium (ethylenimonium) cation derivatives by intramolecular cyclization, which may directly or through formation of a carbonium ion transfer an alkyl group to a cellular nucleophile. Aziridine moiety containing drugs like thio-TEPA act by the same mechanism.

Incorporation of alkylating melphalan (4-[bis{2-chloroethyl}amino]-phenylalanine) into angiotensin II led to a potent competitive antagonist which clearly indicates that the analog did not alkylate the receptor (K.-H. Hsieh and G.R. Marshall, J. Med. Chem. 24 1304-1310, 1981).

Significant cytotoxic activity (inhibition of [$^3$H]thymidine incorporation ) in cultures of human breast cancer cell line T-47D and rat mammary tumor cell line MT-4 and MT-5 was shown with D-melphalan containing LHRH analogs.

Almost all clinically used alkylating agents are bifunctional and have the ability to cross-link two separate molecules, or alkylate one molecule at two separate nucleophilic sites. The cross-links with DNA may be within a single strand, between two complementary strands or between DNA and other molecules, such as proteins. It is thought that the cytotoxicity of alkylating agents correlates with their cross-linking efficiency (J.J. Roberts et al., Adv. Radiat. Biol. 7 211-435, 1978).

Cisplatin (cis-diaminedichloroplatinum) has been used in the cancer therapy for a long time. LHRH analogues with cisplatin related structure in the side-chain have high affinities for membrane receptors of rat pituitary and human breast cancer cells. (S. Bajusz et al. Proc. Natl. Acad. Sci. USA 86 6313-6317, 1989). Incorporation of cytotoxic copper(II) and nickel(II) complexes into suitably modified LHRH analogues resulted in compounds with high hormonal activity and affinity for LHRH receptors on human breast cancer cell membrane. Several of these metallopeptides have cytotoxic activity against human breast and prostate cell lines in vitro, for example pGlu-His-Trp-Ser-Tyr-D-Lys[Ahx-A$_2$bu(SAL)$_2$(Cu)]-Leu-Arg-Pro-Gly-NH$_2$ inhibits the [$^3$H]thymidine incorporation into DNA of the human mammary cell line MDA-MB-231 by 87% at 10 µg dose.

Several antimetabolites are of potential chemotherapeutic interest because of their importance in cellular folate metabolism (I.D. Goldman, et al., Eds. Folyl and Antifolyl Polyglutamates. Plenum press, New York, 1983.). Methotrexate {N-[p[[(2,4-diamino-6-pteridinyl)methyl]methylamino]benzoyl]glutamic acid is a folic acid antagonist that inhibits the function of dihydrofolate reductase and in this way interrupts the synthesis of thymidilate, purine nucleotides, and the amino acids serine and methionine, thereby interfering with the formation of DNA, RNA, and proteins.

SUMMARY OF THE INVENTION

The present invention deals with short, cyclic somatostatin analogues which possess high agonistic activity and comprise cytotoxic moiety incorporated into the peptide sequence or linked to the peptide chain. Cytotoxic moieties, such as nitrogen mustard, antimetabolites, three membered ring moieties, quinones, antitumor antibiotics, platinum complexes, or complexes of metals derived from the non-platinum group metal antitumor agents which are represented by inorganic or organometallic compounds containing transition metals, such as titanium, vanadium, iron, copper, cobalt and gold , and also nickel, cadmium and zinc, or a cytotoxic compound incorporated as a complex of the above mentioned metals.

The compounds of this invention are represented by Formula I

$$Q-R^1-Cys-R^3-D-Trp-Lys-R^6-Cys-R^8-NH_2 \qquad I$$

wherein

$R^1$ is L- or D-Phe, L- or D- Mel,

$R^3$ is Phe if $R^6$ is Thr, or $R^3$ is Tyr if $R^6$ is Val,

$R^8$ is Thr or Trp, and

Q is selected from the group consisting of hydrogen, L-, and D-Mel, methotrexoyl and $AQ^1$ wherein

A is 2,3-diaminopropionyl,

$Q^1$ is $PtCl_2$ or $Cu(B)_2$, and

B is selected from the group consisting of 2-O⁻-1-benzylidenyl or 5-chloro-2-O⁻-1-benzylidenyl,

or the salts thereof with pharmaceutically acceptable acids or bases, with the proviso that when Q is hydrogen $R_1$ is L- or D-Mel

Compounds of Formula I can be prepared by solid phase method or by a combination of the solid phase technique and the classical (solution) synthesis. They are preferably made from intermediate peptides of Formula VIIA

$X^1-R^1-Cys(X^2)-R^3(X^3)-D-Trp-Lys(X^5)-R^6-Cys(X^7)-R^8(X^5)-NH-X^5$     VIIA

wherein

$R^1$, $R^3$, $R^6$, and $R^8$ are as defined above,

$X^1$ is hydrogen or an acyl group of 2-5 carbon atoms, provided that Formula I is an octapeptide or Mel, or Mel-Mel, provided that Formula I is nonapeptide or decapeptide, respectively, or $A(X)_2$, wherein A is as defined above and X are protecting groups on the diamino acid.

$X^2$ and $X^7$ are hydrogens or a protecting group for Cys sulfhydryl group,

$X^3$ is hydrogen or a protecting group for the Tyr phenolic hydroxyl group,

$X^5$ is hydrogen or a protecting group for the Lys $\epsilon$-amino group,

$X^5$ is hydrogen or a protecting group for the Tyr phenolic hydroxyl group,

$X^5$ is hydrogen or benzhydryl group incorporated into a resin.

Compounds of Formula VIIA are intermediates in the process for preparing cyclic compounds of Formula I. The bridge between Cys at positions 2 and 7 is a disulfide bridge (-S-S-). The cyclization reaction carried out after deprotection giving compounds of Formula VIIB and VIIIB:

$$R^1-Cys-R^3-D-Trp-Lys(X^5)-R^6-Cys-R^8-NH_2 \qquad \textbf{VIIB}$$

and

$$A-R^1-Cys-R^3-D-Trp-Lys(X^5)-R^6-Cys-R^8-NH_2 \qquad \textbf{VIIIB}$$

wherein

A, $R^1$, $R^3$, $R^4$, $R^6$, and $R^8$ are as defined above,

4

$X^5$ is hydrogen or a protecting group for the Lys side chain amino group.

The process of preparing metallopeptides with Formula I comprises reacting a peptide of Formula VIIIB (wherein $X^5$ is hydrogen) with $K_2PtCl_4$, wherein $Q^1$ is $PtCl_2$) as cytotoxic moiety, which if desired, can be converted into $PtY_2$ by known methods.

To produce compound of Formula I wherein $Q^1$ is $Cu(B)_2$ comprises of reacting a peptide of Formula VIIIB (wherein $X^5$ is hydrogen) with in situ preformed complex from a hydroxy-oxo Cu compound and a non-platinum-group metal.

A compound of Formula I wherein Q is methotrexoyl can be obtained by alkylation or acylation of compound VIIB (wherein $X^5$ is Boc or FMOC group) with alkyl-or alkanoyl halide, methotrexate.

A pharmaceutical composition is provided by admixing the compound of Formula I with a pharmaceutically acceptable carrier including microcapsules (microspheres) for delayed delivery.

The cytotoxic compounds of present invention have been shown to inhibit the release of growth hormone and supposed to inhibit the release of insulin, glucagon, gastrin, secretin and cholecystokinin as their parent peptides did. Thus, where the growth of tumoral tissue is affected by one of the hormones regulated by the parent peptides, introduction of an additional cytotoxic moiety should enhance the efficiency of a peptide destined to influence hormone sensitive tumours such as prostatic adenocarcinomas, mammary carcinomas, insulinomas, gastrinomas, chondrosarcomas, osteosarcomas, pancreatic ductal, acinar tumors, gastric cancers, colon cancers, certain lung cancers and brain tumors.

## DESCRIPTION OF THE PREFERRED EMBODIMENTS

For convenience in describing this invention, the conventional abbreviations for the amino acids, peptides and their derivatives are used as generally accepted in the peptide art and as recommended by the IUPAC-IUB Commission on Biochemical Nomenclature [European J. Biochem., 138, 9-37 (1984)].

The abbreviations for the individual amino acid residues are based on the trivial name of the amino acid, e.g. pGlu is pyroglutamic acid, His is histidine, Trp is tryptophane, Ser is serine, Tyr is tyrosine, Lys is lysine, Leu is leucine, Arg is arginine, Pro is proline, Gly is glycine, Ala is alanine and Phe is phenylalanine. Where the amino acid residue has isomeric forms, it is the L-form of the amino acid that is represented unless otherwise indicated.

Abbreviations of the uncommon amino acids employed in the present invention are as follows: Mel is 4-[bis(2-chloroethyl)amino]-phenylalanine, $A_2pr$ is 2,3-diaminopropionic acid.

Peptide sequences are written according to the convention whereby the N-terminal amino acid is on the left and the C-terminal amino acid is on the right.

Other abbreviations used are:

| | |
|---|---|
| AcOH | acetic acid |
| $Ac_2O$ | acetic anhydride |
| Boc | tert.butoxycarbonyl |
| Bzl | benzyl |
| CISAL | 5-chloro-2-$O^-$-1-benzylidene |
| DCB | 2,6-dichlorobenzyl |
| DCC | N,N'-dicyclohexylcarbodiimide |
| DCM | dichloromethane |
| DIC | N,N'-diisopropylcarbodiimide |
| DMF | dimethylformamide |
| FMOC | 9 -fluorenylmethyloxycarbonyl |
| HOBt | 1-hydroxybenzotriazole |
| HPLC | high-performance liquid-chromatography |
| MBzl | methylbenzyl |
| MeCN | acetonitrile |
| MeOH | methyl alcohol |
| MTX | methotrexoyl (amethopterin) |
| SAL | 2-$O^-$-1-benzylidene |
| TEA | triethylamine |
| TFA | trifluoroacetic acid |
| Trt | Trityl |
| Z | benzyloxycarbonyl |
| Z(2-Br ) | 2-bromobenzyloxycarbonyl |
| Z(2-Cl) | 2-chlorobenzyloxycarbonyl |

The following peptides are embodiments of the invention:

1. Mel-Cys-Tyr-D-Trp-Lys-Val-Cys-Thr-NH$_2$

2. D-Mel-Cys-Phe-D-Trp-Lys-Thr-Cys-Thr-NH$_2$

3. Mel-D-Phe-Cys-Tyr-D-Trp-Lys-Val-Cys-Thr-NH$_2$

4. Mel-D-Phe-Cys-Phe-D-Trp-Lys-Thr-Cys-Thr-NH$_2$

5. Mel-Phe-Cys-Phe-D-Trp-Lys-Thr-Cys-Thr-NH$_2$

6. Mel-D-Phe-Cys-Tyr-D-Trp-Lys-Val-Cys-Trp-NH$_2$

7. [A$_2$pr(PtCl$_2$)]-D-Phe-Cys-Tyr-D-Trp-Lys-Val-Cys-Thr-NH$_2$

8. [A$_2$pr(PtCl$_2$)]-D-Phe-Cys-Tyr-D-Trp-Lys-Val-Cys-Trp-NH$_2$

9. [A$_2$pr(SAL)$_2$Cu]-D-Phe-Cys-Tyr-D-Trp-Lys-Val-Cys-Thr-NH$_2$

10. [A$_2$pr(ClSAL)$_2$Cu]-D-Phe-Cys-Tyr-D-Trp-Lys-Val-Cys-Thr-NH$_2$

11. MTX-D-Phe-Cys-Tyr-D-Trp-Lys-Val-Cys-Thr-NH$_2$

12. MTX-D-Phe-Cys-Phe-D-Trp-Lys-Thr-Cys-Thr-NH$_2$

Preferred embodiments are peptides No. 1,2,3,5,7,9,11 and 12.

In all of the above embodiments, the compound may also be prepared as the addition salt of pharmaceutically acceptable organic or inorganic acids or bases. As examples, but not as limitations, the following salts may be mentioned: hydrochloride, hydrobromide, sulphate, phosphate, fumarate, gluconate, tannate, maleate, acetate, citrate, benzoate, succinate, alginate, pamoate, malate, ascorbate, tartarate, and the like as well as alkali and alkali earth metal salts.

## Assay procedures

The compounds of this invention exhibit powerful effects on growth hormone (GH) release by the pituitary, bind to tumor cell membranes, inhibit [3H]thymidine incorporation into DNA in cell cultures and the growth of certain tumor cells.

(a) **Growth hormone release inhibiting activities**

The ability of compounds to influence GH and prolactin release in vitro is assayed by using a superfused rat pituitary cell system [S. Vigh and A. V. Schally, Peptides, 5 Suppl. 1, 241-247 (1984)].

To determine hormone release inhibiting effect, each peptide is perfused through the cells for 3 min (1 ml perfusate) in 20-500 pM concentration together with 1 nM GHRH. Rat GH is measured in aliquots of perfusates by radioimmunoassay (with RIA kit from NIAMDDK) to determine either basal or inhibited secretion. The potency of the peptides is compared to that of 25 pM somatostatin (1-14) perfused similarly.

(b) **Receptor binding.**

The affinity for peptides to human breast cancer cell membranes is determined by using $^{125}$I-labelled (Tyr[11])-somatostatin. The binding assay is conducted in a similar manner to that described by M. Fekete et al., J. Clin. Lab. Anal. 3, 137-141, 1989.

(c) **Cytotoxicity test.**

The cytotoxic activity in vitro of compounds in the present invention is measured by a modification of the semiautomated methods of Finlay at al. (Anal. Biochem., 139, 727 (1984)) and Dawson et al. (J. Interferon Res., 6, 137 (1986)). Various human cancer cell lines were cultured in RPMI-164 medium or Dulbecco's modified Eagle's medium. Peptides are added at 1-10,000 ng/ml concentrations and the inhibitory affects on cell growth (fractional % survival) is quantitated by staining cells, solubilizing, and then determining optical density.

The ability of peptides of Formula I to inhibit incorporation of [$^3$H]thymidine into DNA of monolayer cultures the small cell lung cancer line H-69 and MiaPaCa cell line is assayed as described by Kern et al. (Cancer Res., 45, 5436 (1985).

**Synthesis of peptides**

The peptides of the present invention may be prepared by any techniques that are known to those skilled in the peptide art. A summary of the techniques so available may be found in M. Bodanszky, Principles of Peptide Synthesis, Springer-Verlag, Heildelberg, 1984. Classical solution synthesis is described in detail in the treatise "Methoden der Organische Chemie" (Houben-Weyl), Vol. 15, Synthese von Peptiden, Parts I and II, Georg Thieme Verlag, Stuttgart, 1974. The techniques of exclusively solid-phase synthesis are set forth in the textbook of J. M. Stewart and J. D. Young, Solid Phase Peptide Synthesis, Pierce Chem Co., Rockford, IL, 1984 (2nd ed.) and in the review of G. Barany, et al., Int. J. Peptide Protein Res. 30, 705-739, 1987.

The intermediate peptides of this invention and those of the peptides which containing melphalan are synthesized by solid-phase method. In the solid phase synthesis, suitable protected amino acids (sometimes protected peptides) are added stepwise in C--> N direction, once the C-terminal amino acid has been appropriately attached (anchored) to an inert solid support such as a resin. After completion of a coupling step, the N-terminal protecting group is removed from this newly added amino acid residue and the next amino acid, suitably protected, is then added, and so forth. After all the desired amino acids have been linked in the proper sequence, the peptide is cleaved from the support and freed from the remaining protecting group(s) under condition that are minimally destructive towards residues in the sequence. This must be followed by a prudent purification and scrupulous characterization of the synthetic product, so as to ensure that the desired structure is indeed the one obtained.

**Preferred Embodiment of Synthesis**

A particularly preferred method of preparing compounds of Formula I of the present invention is the solid phase synthesis. For example a peptide with Mel in position 0 is prepared by this method as wall as by the classical method as well (by coupling an octapeptide analog with Boc-Mel in solution).

Peptides of Formula I are preferably prepared from intermediate peptides of Formula VIIA:

$$X^1\text{-}R^1\text{-}Cys(X^2)\text{-}R^3(X^3)\text{-}D\text{-}Trp\text{-}Lys(X^5)\text{-}R^6\text{-}Cys(X^7)\text{-}R^8(X^5)\text{-}NH\text{-}X^5 \qquad VIIA$$

wherein

$R^1$, $R^3$, $R^6$, $R^8$ and $X^1$ are as defined hereinabove,
$X^2$ and $X^7$ are methylbenzyl (MBzl) for protecting the sulfhydryl group of Cys,
$X^3$ is 2-Br-benzyloxycarbonyl [Z(2-Br)] or 2,6-dichlorobenzyl (DCB) for protecting the phenolic hydroxyl group of Tyr,
$X^5$ is Z(2-Cl) or FMOC protecting group for side chain amino group of Lys
$X^5$ is 2-Br-benzyloxycarbonyl [Z(2-Br)] or 2,6-dichlorobenzyl (DCB) for protecting the phenolic hydroxyl group of Tyr,
$X^5$ is an amide protecting benzhydryl or methylbenzhydryl group incorporated into resin support; for synthesis of peptide amides, the commercially available benzhydrylamino-polystyrene-2% divinylbenzene copolymer is preferred.

For preparing diaminopropionyl group containing intermediate peptides, the particularly preferred method is the solid phase peptide synthesis. The compounds are obtained by coupling of peptides of Formula VIIA (wherein $X^1$ is hydrogen) with $Boc_2 A_2 pr$, what followed by deprotection gives peptides of the formula

$X_2 A_2 pr$-D-Phe-Cys($X^2$)-Tyr($X^3$)-D-Trp-Lys($X^5$)-Val-Cys($X^7$)-$R^8$($X^5$)-$X^5$ wherein $R^8$, $X^2$, $X^3$, $X^5$, $X^7$, $X^5$ and $X^5$ are as defined above,

X is Boc protecting group for amino groups on $A_2 pr$.

To produce cyclic compounds of Formula VIIB and VIIIB from the reduced Formula VIIA and VIIIA, oxidation of the cysteines two sulfhydryl groups with iodine in dilute acidic solution is the particularly preferred method.

The solid phase synthesis of the peptides of Formula VIIA is commenced by the attachment of Boc-protected Thr or Trp to a benzhydrylamine resin in $CH_2 Cl_2$. The coupling is carried out using DIC or DIC/HOBt at ambient temperature. After the removal of the Boc group, the coupling of successive protected amino acids (each is applied in a 3 molar excess) is carried out in $CH_2 Cl_2$ or in mixtures of $DMF/CH_2 Cl_2$ depending on the solubility of Boc-amino acids. The success of the coupling reaction at each stage of the synthesis is preferably monitored by the ninhydrin test as described by Kaiser et al. [Anal. Biochem. 34, 595 (1970)]. In cases where incomplete coupling occurs, the coupling procedure is repeated before removal of the alpha-amino protecting group prior to the reaction with the next amino acid.

After the desired amino acid sequence of intermediate peptides of Formula VIIA has been completed, the peptide-resin is treated with liquid HF in the presence of anisole to yield the peptides of Formula VIIA wherein $X^2$, $X^3$, $X^5$, $X^7$, $X^5$, and $X^5$ are hydrogens.

Peptides of Formula VIIIB are converted into peptides of Formula I (wherein Q is $AQ^1$ and $Q^1$ is $PtCl_2$) by reacting the peptides with an equivalent amount of $K_2 PtCl_4$ in 60-80% aqueous DMF at ambient temperature followed by purification by HPLC. The platinum containing compounds, if desired, can be converted into their $PtY_2$ containing congeners by known procedures.

The preparation of compounds of Formula I, wherein Q is MTX is carried out by coupling of compound of Formula VIIB with MTX by carbodiimide method.

Peptides of Formula I (wherein Q is $AQ^1$ and $Q^1$ is $Cu(B)_2$) carrying a non-platinum-group metal atom as cytotoxic moiety are obtained from intermediate peptides of Formula VIIIB by coupling with a preformed complex of a hydroxy-oxo compound with a copper cation in 60-80% aqueous DMF solution, then the desired peptide-metal-complex is isolated by HPLC.

Alternatively, compounds of Formula I, wherein Q is $AQ^1$ and $Q^1$ is $Cu(B)_2$ can be prepared from an intermediate peptide of Formula VIIIB (wherein $X^5$ is FMOC protecting group) by reacting it with a suitable hydroxy-oxo- compound, then a metal salt of a pharmaceutically acceptable acid, preferably the acetate of $Cu^{++}$ followed by deprotection with 30-50% of piperidine and isolation by HPLC.

## PURIFICATION OF PEPTIDES

Intermediate peptides of Formula VIIB and Formula VIIIB wherein all of the protecting groups are removed, are purified in two steps. First, the lyophilizate of the solution from the oxidation reaction is subjected to gel filtration on a column (1.2 X 100 cm) of Sephadex G15 fine to remove the polymerized products, the free iodine and salts by elution with 50% of acetic acid. In the second step, the lyophilizate (containing desired peptide) from the gel filtration is purified by high performance liquid chromatography (HPLC) on a reversed phase column. Semipreparative HPLC is carried out on a RAININ HPLC SYSTEM (RAININ Inc., Co., Woburn, MA) consisting of three Rainin Rabbit HP HPLC pumps controlled by an APPLE MACINTOSH PLUS computer, a Rheodyne injector and a KNAUER Model 87 variable wavelength UV monitor. Crude peptides are purified on a DYNAMAX Macro column (21.2 x 250 mm) packed with spherical C18 silicagel (pore size: 300 Å, particle size: 12 $\mu$m) (Rainin Inc., Co.) (**Column A**). Column is eluted with solvent system i consisting of (A) 0.1% aqueous TFA and (B) 0.1% TFA in 70% aqueous acetonitrile. (For preparing the HPLC eluents, UV grade acetonitrile is purchased from Burdick & Jackson, TFA and AcOH are HPLC/Spectro grade (Pierce Chem.) and water is double-distilled in glass and passed through a Milli-Q water purification system (Millipore)).

Melphalan containing peptides of Formula I are also purified in two steps. The melphalan (therefore the melphalan containing peptides) easily undergo decomposition in aqueous solution , therefore the purification should be carried out as a short period of time as possible. To eliminate the oxidized polymers, iodine and salts, a small Sephadex G-15 fine column (0.6 x 10 cm) is applied. To decrease the possibility of the degradation, the purification is carried out at 4 °C. The column is eluted with 90% aqueous AcOH. The

EP 0 450 480 B1

collected fractions are immediately lyophilized, and the peptides are purified by HPLC. A VYDAC Protein & Peptide $C_{18}$ reversed phase column (10 x 250 mm, filled with $C_{18}$ silicagel,pore size: 300 Å, particle size: 5 μm) (ALTECH, Deerfield, IL) (**Column B**) is applied on a same instrument as above. The column is eluted with solvent system ii consisting of (A) 0.2% aqueous acetic acid and (B) 0.2% acetic acid in 70 % aqueous acetonitrile combining isocratic and gradient mode. Column eluent is monitored at 220 nm and fractions are collected in ice-bath.

Platinum containing peptides can be separated in a single HPLC step. Purification is performed on a 10 x 250 mm W-POREX $C_{18}$ column (pore size: 300 Å, particle size: 5 μm) (Phenomenex, Rancho Palos Verdes, CA) (**Column C**) using solvent system i. Chromatography is effected at ambient temperature and column eluate is monitored at 220 nm.

Peptides with Cu cytotoxic moieties need mild conditions at HPLC purification because of the instability of the metal complex in highly acidic solution. Analogues are purified on **Column B** eluted with solvent system iii consisting of (A) 0.1 M ammonium acetate (pH 7.0) and (B) 0.1 M ammonium acetate in 65% aqueous MeCN.

## ANALYTICAL HPLC

The quality and the elution characteristics of crude and purified peptides are established by analytical HPLC on a Hewlett-Packard Model 1090 liquid chromatograph equipped with a diode array detector set a 220 and 280 nm and a reversed phase 4.6 X 250 mm W-POREX $C_{18}$ column (pore size: 300 Å, particle size: 5 μm) (**Column D**). A flow rate of 1.2 ml/min of solvent system i or iii is maintained and the separations are performed at room temperature.

## AMINO ACID ANALYSIS

Peptide samples is hydrolyzed at 110°C for 20 hr in evacuated sealed tubes containing 4M methanesulfonic acid. Analyses were performed with a Beckman 6300 amino acid analyzer.

## MODE OF USE

The peptides of this invention may be used in a preparation of a composition that, if destined for intramuscular or subcutaneous injection, is encased in microcapsules or microparticles formulated from poly (DL-lactide-co-glycolide). Additionally, the peptides may be used for the preparation of a composition that, when destined for intravenous administration, may be dissolved in isotonic saline, phosphate buffer solution, or the like.

If the composition is destined for pharmaceutical application, said composition may contain a peptide of the invention in conjunction with a conventional, pharmaceutically-acceptable carrier. When said composition is destined for intravenous or intramuscular application, the dosage range of the peptide in said composition is from about 1-100 μg of the peptide per kg of the body weight of the host.

These peptides can likewise be used for the preparation of a composition which is to be administered to mammals intravenously, subcutaneously, intramuscularly, intranasally or intravaginally to achieve antitumor effect. Effective dosages of the peptides that are released by said composition will depend from the form of administration of said composition and from the particular species of mammals being treated. An example of one typical dosage form is a physiological saline solution containing the said composition which, after administration, provides a dose of the peptide in the range of about 0.1 to 2.5 mg/kg of body weight.

Although the invention has been described with regard to its preferred embodiments, it should be understood that changes and modifications obvious to one having the ordinary skill in this art may be made without departing from the scope of the invention, which is set forth in the claims which are appended thereto. Substitutions known in the art which do not significantly detract from its effectiveness may be employed in the invention.

# PREPARATION I

D-Phe-Cys-Tyr-D-Trp-Lys-Val-Cys-Thr-NH$_2$      IA

D-Phe-Cys-Tyr-D-Trp-Lys-Val-Cys-Trp-NH$_2$      IB

D-Phe-Cys-Phe-D-Trp-Lys-Thr-Cys-Thr-NH$_2$      IC

Preparation IA is built step by step on a benzhydrylamine HCl resin containing about 0.55 meq NH$_2$/g (Advanced ChemTech, Louisville, KY) in a reaction vessel for manual solid-phase synthesis. 0.5g benzhydrylamine HCl resin (about 0.27 mmol) is neutralized with 10% triethylamine in CH$_2$Cl$_2$ two times each for three minutes and washed with CH$_2$Cl$_2$ six times. The resin is mixed with three molar excess of Boc-Thr(Bzl) (0.75 mmoles) and HOBt (0.82 mmoles) in DMF for three minutes. 5% diisopropylcarbodiimide (0.82 mmoles) in CH$_2$Cl$_2$ is added. The mixture is shaken at room temperature for 90 min. The resulting resin is washed with DCM six times and subjected to a Kaiser test (Kaiser et al., Anal. Biochem. 34, 595 (1970)).

The removal of the Boc- group (deprotection) from Boc-Thr(Bzl)-BHA resin is carried out with a solution of trifluoroacetic acid in DCM (1:1) for 5 minutes, filtered and treated again for 25 min, filtered and then washed with DCM six times.

Neutralization with 10% triethylamine is performed as described for the benzhydrylamine resin.

The subsequent amino acid residues (Boc-Cys(MBzl), Boc-Val, Boc-Lys[Z2-Cl)], Boc-D-Trp, Boc-Tyr[Z-(2-Br)], Boc-Cys(MBzl) and Boc-D-Phe) are then introduced sequentially by coupling in the same manner as described above to yield a peptide resin with a structure of Boc-D-Phe-Cys(MBzl)-Tyr[Z(2-Br)]-D-Trp-Lys[Z-(2-Cl)]-Val-Cys(MBzl)-Thr(Bzl)-BHA resin.

The deprotection is performed as described for Boc-Thr(Bzl)-BHA resin. After incorporating Boc-D-Trp, 5% mercaptoethanol is added to 50% trifluoroacetic acid in DCM.

Finally, the Boc-deprotected peptide resin is washed with DCM, methanol and DCM three times each and dried under vacuum.

500 mg protected octapeptide BHA resin is mixed with 0.5 ml anisole and stirred in 10 ml liquid HF at 0°C for 1 hour. After elimination of HF under vacuum, the peptide and resin mixture is washed with dry ethylacetate. The peptide is then extracted with 30% AcOH, separated from the resin by filtration, and lyophilized.

100 mg of crude, reduced peptide is dissolved in 100 ml 95% aqueous AcOH, then 0.005 M iodine solution in glacial acetic acid is dropped in until the yellow color persists. After evaporation of the acetic acid, the crude, disulfide bridge containing peptide is subjected to gel filtration on Sephadex G15 fine column. Further purification is performed by semipreparative HPLC on **Column A** using solvent system, i with a linear gradient at flow rate of 5.0 ml/min.

Preparation of peptide

D-Phe-Cys-Tyr-D-Trp-Lys-Val-Cys- Trp-NH$_2$

(Preparation IB) is accomplished as described above with the exception that Boc-Trp is anchored to the resin instead of Boc-Thr(Bzl) in the first step.

D-Phe-Cys-Tyr-D-Trp-Lys-Val-Cys-Trp-NH$_2$

(1C) is synthesized by method related the syntheses for Preparation IA except that Boc-Thr(Bzl) is incorporated in the third coupling step instead of Boc-VAl.

Purified peptides (IA, IB, IC,) (28 mg, 21 mg, 32 mg,) proved to be pure (>95%) in analytical HPLC using solvent system i in a linear gradient mode, and the amino acid analysis give the expected results.

| Peptide | | Gradient (%B/min) for | | Retention time |
| No. | Code | Purification | Analysis | on Column D (Min) |
| IA | | 25-55/60 | 20-60/40 | 15.5 |
| IB | | 30-60/60 | 25-65/40 | 19.5 |
| IC | | 25-55/60 | 20-60/40 | 17.4 |

## PREPARATION II

D-Phe-Cys-Tyr-D-Trp-Lys(FMOC)-Val-Cys-Thr-NH$_2$    IIA

D-Phe-Cys-Tyr-D-Trp-Lys(FMOC)-Val-Cys-Trp-NH$_2$    IIB

D-Phe-Cys-Phe-D-Trp-Lys(FMOC)-Thr-Cys-Thr-NH$_2$    IIC

Preparation IIA is built step by step on BAH resin in accordance with the method described for Preparation IA except that Boc-Lys(FMOC) is used in place of Boc-Lys[Z(2-Cl)]. The peptide is prepared in eight successive coupling step giving Boc-D-Phe-Cys(MBzl)-Tyr[Z(2-Br)]-D-Trp-Lys(FMOC)-Val-Cys(MBzl)-Thr(Bzl)-BHA resin. Boc-deprotected peptide-resin is then treated with HF/anisole, oxidized with iodine and purified on a Sephadex G-15 column and on **Column A** eluted with solvent system i.

| Peptide | | Gradient (%B/min) for | | Retention time |
| No. | Code | Purification | Analysis | on Column D (Min) |
| IIA | | 55-85/60 | 50-90/40 | 12.2 |
| IIB | | 55-85/60 | 50-90/40 | 14.6 |
| IIC | | 50-80/60 | 50-90/40 | 12.0 |

## PREPARATION III

A$_2$pr-D-Phe-Cys-Tyr-D-Trp-Lys-Val-Cys-Thr-NH$_2$    IIIA

A$_2$pr-D-Phe-Cys-Tyr-D-Trp-Lys-Val-Cys-Trp-NH$_2$    IIIB

Peptide IIIA is prepared by the solid phase technique on BAH resin (1.0 g, 0.55 meq NH$_2$) following the same procedures set forth for Preparation I, except that the synthesis doesn't stop with the addition of D-Phe. The octapeptide is acylated with (Boc)$_2$A$_2$pr to yield 391 mg of (Boc)$_2$-A$_2$pr-D-Phe-Cys(MBzl)-Tyr[Z(2-Br)]-D-Trp-Lys[Z(2-Cl)]-Val-Cys(MBzl)-Thr(Bzl)-BHA resin. After removing the Boc groups, peptide-resin is treated with HF/anisole to afford the free, reduced peptide. Oxidation followed by purification on Sephadex G-15 fine column and by HPLC on **Column A** with a gradient of solvent system i (20-50 %B in 60 min) yields Preparation IIIA (86 mg).

Proceeding in a similar manner but using Boc-Trp in place of Boc-Thr(Bzl) in the first step of the synthesis, yields Preparation IIIB (77 mg).

Pure peptides (>94%) having HPLC retention time 14.5 min and 23.7 min, respectively, when using solvent system i in linear gradient mode(20-50 %B in 30 min), have the expected amino acid composition.

# PREPARATION IV

$$A_2pr-D-Phe-Cys-Tyr-D-Trp-Lys(FMOC)-Val-Cys-Thr-NH_2 \qquad IV$$

The synthesis of Preparation IV is accomplished as described at Preparation I except that Boc-Lys-(FMOC) is used in the fourth coupling step in place of Boc-Lys[Z-(2-Cl)]. Performing a similar HF cleavage, oxidation and purification procedure (**Column A**, solvent system i, gradient elution : 50-80 %B in 60 min) gives Preparation IV (47 mg). Analytical HPLC of the pure peptide shows one peak with retention time 12.5 min.

# PREPARATION V

$$D-Phe-Cys-Tyr-D-Trp-Lys(Boc)-Val-Cys-Thr-NH_2 \qquad VA$$

$$D-Phe-Cys-Tyr-D-Trp-Lys(Boc)-Val-Cys-Trp-NH_2 \qquad VB$$

Preparation VA was synthesized as described for Preparation IA with the exception that FMOC-D-Phe was used in place of Boc-D-Phe. The peptide-resin was treated with HF/anisol, then disulfide bridge was formed by oxidation and the peptide was purified on Sephadex G-15 fine column and by HPLC (Column A, solvent system i, gradient elution 55-85% B in 60 min). The FMOC-protected peptide was dissolved in DMF and 1.2 equivalents of di-tert-butyl dicarbonate and 1.2 equivalent TEA were added to produce Boc-Lys($\epsilon$-Boc)- containing peptide. When the reaction was complete, the protected peptide was precipitated with ether and the FMOC group was removed with 50% piperidine. The Boc-protected peptide was chromatographed on Column A with solvent system i (45-75%B in 60 min) to give Preparation VA.

Proceeding in a similar manner but using Boc-Trp in place of Boc-Thr(Bzl), the reaction affords Preparation VB. The peptides proved to be pure on analytical HPLC using solvent system i (45-85%B in 40 min).

# PREPARATION VI

$$Glt-D-Phe-Cys-Tyr-D-Trp-Lys(FMOC)-Val-Cys-Thr-NH_2 \qquad VI$$

Preparation VI was produced by following the snythesis of IIA by acylation of free N-terminal amino group with glutaric anhydride. The peptide-resin was treated with HF/anisol, disulfide bridge was formed by oxidation, purified on Sephadex G-15 fine column and finally by HPLC (Column A, solvent i, gradient elution: (50-80%B in 60 min).

**EXAMPLE 1**

$$\text{Mel-Cys-Tyr-D-Trp-Lys-Val-Cys-Thr-NH}_2 \qquad 1$$

$$\text{D-Mel-Cys-Phe-D-Trp-Lys-Thr-Cys-Thr-NH}_2 \qquad 2$$

Peptide 1 is prepared by successive coupling of Boc-Thr(Bzl), Boc-Cys(MBzl), Boc-Val, Boc-Lys[Z(2-Cl)],Boc-D-Trp, Boc-Tyr[Z(2-Br)], Boc-Cys(MBzl) and Boc-Mel amino acids to BAH resin (100 mg) in a same manner as described for Preparation I. Step by step coupling of Boc-Thr(Bzl), Boc-Cys(MBzl), Boc-Val, Boc-Lys[Z(2-Cl)],Boc-D-Trp, Boc-Tyr[Z(2-Br)], Boc-Cys(MBzl) and Boc-D-Mel to BAH resin (100 mg) led to Peptide 2-resin. After cleavage of peptides from the resin, they are oxidized and lyophilized, then subjected to gel filtration on a small Sephadex G-15 column washed with 80% aqueous AcOH to remove the iodine. Purification by HPLC on **Column B** with solvent system ii using gradient elution (55-85 B% in 60 min) gives Peptide 1 (13 mg) and Peptides 2 (14 mg) with purity >95% having HPLC retention time 20.1 min and 22.8 min, respectively, when analytical HPLC is carried out using solvent system i (30-70 %B in 40 min).

**EXAMPLE 2**

$$\text{Mel-D-Phe-Cys-Tyr-D-Trp-Lys-Val-Cys-Thr-NH}_2 \qquad (3)$$

$$\text{Mel-D-Phe-Cys-Phe-D-Trp-Lys-Thr-Cys-Thr-NH}_2 \qquad (6) \ (4)$$

$$\text{Mel-Phe-Cys-Phe-D-Trp-Lys-Thr-Cys-Thr-NH}_2 \qquad (7) \ (5)$$

$$\text{Mel-D-Phe-Cys-Tyr-D-Trp-Lys-Val-Cys-Trp-NH}_2 \qquad (9) \ (6)$$

D-Phe-Cys(MBzl)-Tyr[Z(2-Br)]-D-Trp-Lys[Z(2-Cl)]-Val-Cys(MBzl)-Thr(Bzl)-BAH resin is synthesized as Preparation I and after N-terminal deprotection it is acylated with Boc-Mel giving protected nonapeptide 3.

Coupling Boc-Thr(Bzl), Boc-Cys(MBzl), Boc-Thr(Bzl), Boc-Lys[Z(2-Cl)], Boc-D-Trp, Boc-Phe, Boc-Cys-(MBzl), Boc-D-Phe and Boc-Mel sequentially to benzhydryl amine resin as described at Preparation I gives protected nonapeptide 4.

Successive coupling of Boc-Thr(Bzl), Boc-Cys(MBzl), Boc-Thr(Bzl), Boc-Lys[Z(2-Cl)], Boc-D-Trp, Boc-Phe, Boc-Cys(MBzl), Boc-Phe and Boc-Mel to BAH resin led to Peptide 5-resin.

Mel-D-Phe-Cys(Bzl)-Tyr[Z(2-Br)]-D-Trp-Lys[Z(2-Cl)]-Val-Cys(MBzl)-Trp-BAH resin (protected Peptide 6) is produced in nine coupling steps according to the method described for Preparation I.

Applying similar cleavage, oxidation and gel filtration on a small Sephadex G-15 column as in Example 1, Peptide 3, 4, 5 and 6 are isolated (8.4 mg, 10.6 mg, 9.9 mg, 7.9 mg, respectively) by HPLC on **Column B** using solvent system ii with purity >92%.

## HPLC DATA

| Peptide | | Gradient (%B/min) for | | Retention time |
| No. | Code | Purification | Analysis | on Column D (Min) |
| --- | --- | --- | --- | --- |
| 3 | | 60-90/60 | 40-80/40 | 16.1 |
| 4 | | 60-90/60 | 40-80/40 | 14.0 |
| 5 | | 69-90/60 | 35-75/40 | 17.8 |
| 6 | | 65-95/60 | 40-80/40 | 22.4 |

To prove the possibility of applying solid phase peptide syntheses to incorporate instable melphalan into the sequence, Peptide 3 is prepared by another method as well.

FMOC-D-Phe-Cys-Tyr-D-Trp-Lys-Val-Cys-Thr-NH$_2$

is synthesized as described for Preparation IA with the exception that FMOC-D-Phe is used in place of Boc-Phe. Boc-Mel is coupled to this octapeptide applying the classical synthesis method.

FMOC-D-Phe-Cys-Tyr-D-Trp-Lys-Val-Cys-Thr-NH$_2$

| Boc$_2$O
↓

FMOC-D-Phe-Cys-Tyr-D-Trp-Lys(Boc)-Val-Cys-Thr-NH$_2$

| 50% piperidine in DMF
↓ for 30 min

D-Phe-Cys-Tyr-D-Trp-Lys(Boc)-Val-Cys-Thr-NH$_2$

| coupling with Boc-Mel
↓ DCC + HOBt

Boc-Mel-D-Phe-Cys-Tyr-D-Trp-Lys(Boc)-Val-Cys-Thr-NH$_2$

| deprotection with
↓ 50% TFA in DCM

(3)    Mel-D-Phe-Cys-Tyr-D-Trp-Lys-Val-Cys-Thr-NH$_2$

Peptide 3 synthesized by this way has identical amino acid composition, UV spectra and HPLC retention time as those that prepared on solid phase.

**EXAMPLE 3**

$$\overbrace{[A_2pr(PtCl_2)]-D-Phe-Cys-Tyr-D-Trp-Lys-Val-Cys}-Thr-NH_2 \qquad (7)$$

$$\overbrace{[A_2pr(PtCl_2)]-D-Phe-Cys-Tyr-D-Trp-Lys-Val-Cys}-Trp-NH_2 \quad (13) \quad (8)$$

Platinum containing Peptide 7 is prepared by reacting 30 mg of

$$\overbrace{A_2pr-D-Phe-Cys-Tyr-D-Trp-Lys-Val-Cys}-Thr-NH_2$$

intermediate peptideTFA salt (Preparation IIA) (dissolved in 100 $\mu$l DMF and neutralized with NaOH) with potassium chloroplatinate (8.4 mg) in 100 $\mu$l water for 48 hours in the presence of 4 mg sodium acetate. Thereafter the reaction mixture diluted with water and injected onto **Column C** and chromatographed using solvent system i (25-50 %B in 50 min).

Peptide 8 is synthesized in similar manner, except that

$$\overbrace{A_2pr-D-Phe-Cys-Tyr-D-Trp-Lys-Val-Cys}-Trp-NH_2$$

trifluoroacetic acid salt (31 mg, Preparation IIB) is used as starting material instead of the corresponding Thr[8] derivative (Preparation IIA).

The ensuing metallopeptides (4.8 mg, 5.3 mg) show one peak in HPLC with retention times 9.1 min and 19.5 min, respectively, eluted with solvent system i (30-60 %B in 30 min).

**EXAMPLE 4**

$$\overbrace{[A_2pr(SAL)_2Cu]-D-Phe-Cys-Tyr-D-Trp-Lys-Val-Cys}-Thr-NH_2 \quad (9)$$

$$\overbrace{[A_2pr(ClSAL)_2Cu]-D-Phe-Cys-Tyr-D-Trp-Lys-Val-Cys}-Thr-NH_2 \quad (10)$$

Metallopeptide 9 is produced in two different method to prove that the desired Cu complex can be prepared despite of the presence of free Lys $\epsilon$-amino group.

In method A,

$$\overbrace{A_2pr-D-Phe-Cys-Tyr-D-Trp-Lys(FMOC)-Val-Cys}-Thr-NH_2$$

(Preparation III) is dissolved in 100 $\mu$l DMF, the pH is adjusted to 8 with NaOH and NaOAc and then 4 mg salicylaldehyde is added. After standing at room temperature for 1 hour, the metal complex is composed by reacting with copper(II)acetate (3mg in 50 $\mu$l water) for 30 min. To remove FMOC protecting group, the reaction mixture is mixed with 50 $\mu$l piperidine, and after 30 min, it is diluted with 100 $\mu$l water. The precipitate is centrifuged, the green supernatant solution is injected onto **Column C** and eluted with solvent system ii (35-55 %B in 40 min) to isolate the metallopeptide 9 (4.1 mg). Testing on **Column D** with solvent system ii (40-85 %B in 30 min) it proves to be pure and its retention time is 11.2 min.

According to method B, 30 mg of free intermediate peptide (Preparation IIA) is reacted with 7.4 mg preformed bis(salicylaldehydato)copper (II) [Y.Nakao and A. Nakahara, Bull. Chem. Soc. Japan 46, 187

15

(1973)] in 300 $\mu$l 90 % aqueous DMF containing sodium acetate (2 mg) for 48 hours. Peptide-SAL-Cu complex is separated on **Column C** using solvent system iii (40-60 %B in 40 min). Peptide 9 obtained (7.6 mg) by this method has same retention time and UV spectrum as that prepared by method A.

Metallopeptide 10 with CISAL is produced by method B: 16 mg of intermediate peptide TFA salt (Preparation IIA) is allowed to react with 3.9 mg bis(5-chloro-salicylaldehydato)copper(II) in NaOAc buffered aqueous DMF solution for 48 hours. The reaction mixture is chromatographed on **Column C** eluted with solvent system iii (45-70% B in 50 min). The isolated greenish peptide (4.1 mg) has HPLC retention time 11.4 min, when tested on **Column D** with solvent system ii in linear gradient mode (45-90 %B in 30 min).

**EXAMPLE 5**

$$\text{MTX-D-Phe-Cys-Tyr-D-Trp-Lys-Val-Cys-Thr-NH}_2 \qquad (11)$$

$$\text{MTX-D-Phe-Cys-Phe-D-Trp-Lys-Thr-Cys-Thr-NH}_2 \qquad (12)$$

Synthesis of antimetabolite containing Peptide 11 is performed by acylating

$$\text{D-Phe-Cys-Tyr-D-Trp-Lys(FMOC)-Val-Cys-Thr-NH}_2$$

(Preparation IIA) with methotrexate. To the solution of 5.6 mg methotrexate in 100 $\mu$l DMF, equivalent amount of diisopropyl-carbodiimide is added at 0°C. After 15 min it is mixed with neutralized solution (DMF) of 14 mg peptide and is kept 0°C overnight. FMOC protecting group is removed by treating with 50 $\mu$l piperidine, then the peptide is purified on **Column C** eluted with solvent system i (25-50 %B in 50 min). Two peptides are detected (and isolated) (retention times are 14.9 and 15.4 min), corresponding to the acylation with $\alpha$-carboxyl or -carboxyl group of methotrexate, when tested on **Column D** in solvent system i (30-50 %B in 20 min).

Peptide 13 is prepared in a same manner except that

$$\text{D-Phe-Cys-Phe-D-Trp-Lys(FMOC)-Thr-Cys-Thr-NH}_2$$

(Preparation IIC) is used as a starting material.

**EXAMPLE 6**

Biological effects, receptor binding potencies and cytotoxic activities of peptides of this invention are summarized in Table 1-4.

Table 1 shows the growth hormone release inhibiting activity of the compounds of this invention as compared to that of somatostatin(1-14) in dispersed rat pituitary cell superfusion system in vitro [S. Vigh and A. V. Schally, Peptides 5, 241-247 (1984)]. Table 1 also contains data on the receptor binding affinity of these compounds for rat cortex and rat prostate tumor (Dunning R3327H) cell membranes.

Data in Table 2 show the effect of Peptide 5 on the growth of 3T3 fibroblast cells as measured by spectrophotometric method. $10^5$ cells are grown in 10% NCS in DME in 96 well plates pretreated with poly-D-lysine to allow the cells to adhere firmly. The cells are treated for 3 days with daily changes of the cytotoxic peptides at 1-10,000 ng/ml concentrations. Following the incubation period the metabolic dye MTT (3-[4,5-dimethylthiazol-2-yl]-2,5-diphenyl-2H-tetrazolium bromide) is added and after the development of the purple formazan color by live cells, the absorbance is measured at 590 nm (4th day). Previous standardization with a known number of cells relates absorbance to actual live cell number.

Table 3 shows data on the inhibition of $^3$H-thymidine incorporation into DNA by somatostatin analogues on cultured human pancreatic MiaPaCa cancer cell line. $1 \times 10^5$ cells are plated with DME media containing 10% fetal calf serum (FCS) on Day 0. After 24 hours media is removed and replaced with DME media without serum and incubated at 37°C for 2 days. Media was then removed and replaced with DME media containing 5% FCS with or without somatostatin analog. Media with peptides was changed daily for 4 days. On day 4, 1 $\mu$Ci $^3$H-thymidine added and incubated an additional 17 hrs. Media is then removed and cells recovered by trypsination. DNA extracted with 1 N perchloric acid and the radioactivity measured.

Table 4 contains data on the inhibition of $^3$H-thymidine incorporation into DNA in H-69 small cell lung cancer cell line by somatostatin analogs. $6.5 \times 10^4$ cell are plated in sterile polypropylene tubes in RPMI-1640 containing 10% horse serum and 5% fetal calf serum. After three days somatostatin analogs are added and incubated five days at 37°C. On day 4, 1 $\mu$Ci of tritiated thymidine is added and of day 5, cells are washed and collected on glass paper, washed with 10% aqueous trichloroacetic acid, ethyl alcohol and counted in liquid scintillation counter.

TABLE 1

| GH-release inhibiting activity and receptor binding affinity of somatostatin analogues containing cytotoxic moiety. | | | |
|---|---|---|---|
| No. of Peptide | Inhibiting activity (%) | Affinity constant**$(K_a \times 10^9 M^{-1})$ | |
| | | rat cortex | Dunning tumor |
| SS-14 | 12.0 | 15.795 | 1.378 |
| 1. | 26.0 | 13.355 | 0.188 |
| 3. | | 18.790 | N.B. |
| 4. | | N.B. | 0.111 |
| 6. | | 5.371 | 0.694 |
| 7. | 44.6 | 49.529 | 1.947 |
| 8. | | N.B. | 0.115 |
| 9. | 7.3 | 166.035 | 0.004 |
| 10. | 38.5 | 4.027 | 0.277 |
| 11A. | 30.5 | 11.396 | 0.642 |
| 11B. | 26.4 | 3.287 | 9.589 |

*GH-release inhibiting activity is checked at dose 200nM.
**[$^{125}$I-Tyr[11]] somatostatin 1-14 used as labelled ligand.
N. B. no binding

TABLE 2

| Effect of Peptide 5 on the growth of 3T3 fibroblast cells as measured by the MTT spectrophotometric assay. | | | |
|---|---|---|---|
| Compound | Dose | Absorbance | % Decrease |
| Control | -- | 0.252 | 0 |
| Peptide 5 | 10 | 0.125 | 50* |
| | 100 | 0.192 | 24* |
| | 100 | 0.206 | 18* |
| | 1000 | 0.186 | 26* |
| | 10000 | 0.121 | 52* |

*p< 0.01 by Duncan's multiple range test.

EP 0 450 480 B1

TABLE 3

| Effect of Peptide 5 and melphalan on the growth of MiaPaCa cells as measured by cell counts and $^3$H-thymidine incorporation into DNA. | | | |
|---|---|---|---|
| Compound | Dose | % Decrease in cell number | % inhibition of $^3$H-thymidine incorp. |
| Control | 0 | -- | -- |
| Peptide 5 | $10^{-6}$ M<br>$10^{-7}$ M<br>$10^{-8}$ M | 22**<br>33**<br>35** | 68**<br>63**<br>53** |
| Melphalan | $10^{-6}$ M<br>$10^{-7}$ M<br>$10^{-8}$ M | 29**<br>1<br>1 | 20<br>18<br>-- |

*p< 0.05 by Duncan's multiple range test.
**p< 0.01

TABLE 4

| Effect of Peptide 5 on the incorporation of $^3$H-thymidine into DNA in SCLC line H-69. | | | |
|---|---|---|---|
| Compound | Dose | % inhibition $^3$H-thymidine incorp. | of p* |
| Control | 0 | 0 | -- |
| Peptide 5 | 1<br>10<br>100<br>1000<br>10000 | 37<br>34<br>25<br>29<br>36 | 0.01<br>0.01<br>0.01<br>0.01<br>0.01 |

* significance determined by Duncan's multiple range test.

**Claims**

1. A peptide of the formula:

$$\text{Q} - \text{R}^1 - \text{Cys} - \text{R}^3 - \text{D} - \text{Trp} - \text{Lys} - \text{R}^6 - \text{Cys} - \text{R}^8 - \text{NH}_2$$

wherein
$R^1$ is L- or D-Phe, L- or D- Mel,
$R^3$ is Phe if $R^6$ is Thr, or $R^3$ is Tyr if $R^6$ is Val,
$R^8$ is Thr or Trp, and
Q is selected from the group consisting of hydrogen, L-, and D-Mel, methotrexoyl and $AQ^1$
wherein
A is 2,3-diaminopropionyl,
$Q^1$ is $PtCl_2$ or $Cu(B)_2$, and
B is selected from the group consisting of 2-O$^-$-1-benzylidenyl or 5-chloro-2-O$^-$-1-benzylidenyl,
or the salts thereof with pharmaceutically acceptable acids or bases, with the proviso that when Q is hydrogen $R^1$ is L- or D-Mel.

2. A peptide of claim 1 wherein $R^1$ is D-Mel, $R^8$ is Thr and Q is hydrogen and $R^3$ is Phe.

18

3. A peptide of claim 1 wherein Q is Mel, $R^8$ is Thr and $R^1$ is Phe.

4. A peptide of claim 1 wherein $R^1$ is D-Phe, $R^3$ is Phe and Q is methotrexoyl.

5. A peptide of claim 1 wherein Q is hydrogen, $R^1$ is Mel, $R^3$ is Tyr and $R^8$ is Thr.

6. A peptide of claim 1 wherein $R^1$ is D-Phe, $R^3$ is Tyr and Q is Mel.

7. A peptide of claim 1 wherein $R^1$ is D-Phe, $R^3$ is Tyr Q is methotrexoyl and $R^8$ is Thr.

8. A peptide of claim 1 wherein $R^1$ is D-Phe, $R^3$ is Tyr, $R^8$ is Thr and Q is 2,3-diaminopropionyl-$PtCl_2$ or 2,3-diaminopropionyl-$Cu(B)_2$ wherein B is selected from the group consisting of $2\text{-}O^-\text{-}1$-benzylidenyl or $5\text{-chloro-}2\text{-}O^-\text{-}1$-benzylidenyl.

9. Use of any of the peptides according to claims 1 through 8 in a preparation of a compound destined to influence growth of cancerous tumors.

**Patentansprüche**

1. Peptid der Formel:

$$Q\text{--}R^1\text{--}Cys\text{--}R^3\text{--}D\text{--}Trp\text{--}Lys\text{--}R^6\text{--}Cys\text{--}R^8\text{--}NH_2$$

in der
$R^1$ L- oder D-Phe, L- oder D-Mel bedeutet,
$R^3$ Phe bedeutet, wenn $R^6$ Thr bedeutet, oder $R^3$ Tyr bedeutet, wenn $R^6$ Val bedeutet,
$R^8$ Thr oder Trp bedeutet und
Q ausgewählt ist aus der Gruppe, bestehend aus Wasserstoff, L- und D-Mel, Methotrexoyl und $AQ^1$, worin
    A 2,3-Diaminopropionyl bedeutet,
    $Q^1$ $PtCl_2$ oder $Cu(B)_2$ bedeutet und
    B ausgewählt ist aus der Gruppe, bestehend aus $2\text{-}O^-\text{-}1$-Benzylidenyl oder $5\text{-Chlor-}2\text{-}0^-\text{-}1$-Benzylidenyl,
oder die Salze desselben mit pharmazeutisch akzeptablen Säuren oder Basen, mit der Bedingung , daß $R^1$ L- oder D-Mel ist, wenn Q Wasserstoff ist.

2. Peptid nach Anspruch 1, bei dem $R^1$ D-Mel, $R^8$ Thr, Q Wasserstoff und $R^3$ Phe bedeutet.

3. Peptid nach Anspruch 1, bei dem Q Mel, $R^8$ Thr und $R^1$ Phe bedeutet.

4. Peptid nach Anspruch 1, bei dem $R^1$ D-Phe, $R^3$ Phe und Q Methotrexoyl bedeutet.

5. Peptid nach Anspruch 1, bei dem Q Wasserstoff, $R^1$ Mel, $R^3$ Tyr und $R^8$ Thr bedeutet.

6. Peptid nach Anspruch 1, bei dem $R^1$ D-Phe, $R^3$ Tyr und Q Mel bedeutet.

7. Peptid nach Anspruch 1, bei dem $R^1$ D-Phe, $R^3$ Tyr, Q Methotrexoyl und $R^8$ Thr bedeutet.

8. Peptid nach Anspruch 1, bei dem $R^1$ D-Phe, $R^3$ Tyr, $R^8$ Thr und Q 2,3-Diaminopropionyl-$PtCl_2$ oder 2,3-Diaminopropionyl-$Cu(B)_2$ bedeutet, wobei B ausgewählt ist aus der Gruppe, bestehend aus $2\text{-}O^-\text{-}1$-Benzylidenyl oder $5\text{-Chlor-}2\text{-}O^-\text{-}1$-benzylidenyl.

9. Verwendung von einem der Peptide nach Anspruch 1 bis 8 bei einer Herstellung von einer Verbindung, die zum Beeinflussen des Wachstums von krebsartigen Tumoren bestimmt ist.

**Revendications**

1.  Peptide de formule :

$$Q-R^1-Cys-R^3-D-Trp-Lys-R^6-Cys-R^8-NH_2$$

dans lequel :

$R^1$ est L- ou D-Phe, L- ou D-Mel;

$R^3$ est Phe si $R^6$ est Thr, ou $R^3$ est Tyr si $R^6$ est Val;

$R^8$ est Thr ou Trp, et

Q est choisi parmi le groupe consistant en hydrogène, L- et D-Mel, méthotrexoyle et $AQ^1$

où :

A est 2,3-diaminopropionyle;

$Q^1$ est $PtCl_2$ ou $Cu(B)_2$, et

B est choisi parmi le groupe consistant en $2-O^{(-)}-1$-benzylidènyle ou $5$-chloro-$2-O^{(-)}-1$-benzylidènyle,

ou les sels de ceux-ci avec des acides ou des bases pharmaceutiquement acceptables, à la condition que lorsque Q est hydrogène, $R^1$ est L- ou D-Mel.

2.  Peptide suivant la revendication 1, dans lequel :

$R^1$ est D-Mel;

$R^8$ est Thr;

Q est hydrogène, et

$R^3$ est Phe.

3.  Peptide suivant la revendication 1, dans lequel :

Q est Mel;

$R^8$ est Thr, et

$R^1$ est Phe.

4.  Peptide suivant la revendication 1, dans lequel :

$R^1$ est D-Phe;

$R^3$ est Phe, et

Q est méthotrexoyle.

5.  Peptide suivant la revendication 1, dans lequel :

Q est hydrogène;

$R^1$ est Mel;

$R^3$ est Tyr, et

$R^8$ est Thr.

6.  Peptide suivant la revendication 1, dans lequel :

$R^1$ est D-Phe;

$R^3$ est Tyr, et

Q est Mel.

7.  Peptide suivant la revendication 1, dans lequel :

$R^1$ est D-Phe;

$R^3$ est Tyr;

Q est méthotrexoyle, et

$R^8$ est Thr.

8. Peptide suivant la revendication 1, dans lequel :

   $R^1$ est D-Phe;

   $R^3$ est Tyr;

   $R^8$ est Thr, et

   Q est 2,3-diaminopropionyl-PtCl$_2$ ou 2,3-diaminopropionyl-Cu(B)$_2$, où B est choisi parmi le groupe consistant en 2-O$^{(-)}$-1-benzylidènyle ou 5-chloro-2-O$^{(-)}$-1-benzylidènyle.

9. Utilisation de l'un quelconque des peptides suivant les revendications 1 à 8, dans la préparation d'un composé destiné à influencer la croissance des tumeurs cancéreuses.